# EUROPEAN PATENT SPECIFICATION

(11) **EP 3 961 828 B1**
(45) Date of publication and mention of the grant of the patent: **11.10.2023**
(21) Application number: 20796325.7
(22) Date of filing: 19.02.2020
(51) Int. Cl.: C09K 11/77, H01S 5/022, A61B 1/00, A61B 1/06, A61B 1/07, G02B 21/06, G02B 23/26, H01L 33/50, H01S 5/00, A61N 5/06, A61B 1/04, G02B 21/00, G02B 23/24, G02B 26/00, H01L 25/075

(54) **LIGHT EMITTING DEVICE; AND MEDICAL SYSTEM, ELECTRONIC APPARATUS, AND INSPECTION METHOD USING SAME**
LICHTEMITTIERENDE VORRICHTUNG UND MEDIZINISCHES SYSTEM, ELEKTRONISCHES GERÄT UND INSPEKTIONSVERFAHREN DAMIT
DISPOSITIF ÉLECTROLUMINESCENT ET SYSTÈME MÉDICAL, APPAREIL ÉLECTRONIQUE ET PROCÉDÉ D'INSPECTION L'UTILISANT

(30) Priority: 24.04.2019 JP 2019082915
(43) Date of publication of application: 02.03.2022
(73) Proprietor: Panasonic Intellectual Property Management Co., Ltd., Osaka-shi, Osaka 540-6207 (JP)
(72) Inventor: ABE, Takeshi, Osaka-shi, Osaka 540-6207 (JP); OSHIO, Shozo, Osaka-shi, Osaka 540-6207 (JP); NITTA, Mitsuru, Osaka-shi, Osaka 540-6207 (JP)
(74) Representative: Müller-Boré & Partner Patentanwälte PartG mbB
(86) International application number: PCT/JP2020/006522
(87) International publication number: WO 2020/217669

(56) References cited:
- EP-A1- 3 904 488
- WO-A1-2018/143198
- WO-A1-2018/207703
- WO-A1-2018/207703
- JP-A- 2006 114 911
- JP-A- 2007 017 986
- JP-A- 2009 226 072
- JP-A- 2012 104 245
- JP-A- 2018 515 913
- JP-A- 2018 518 046
- JP-B1- 6 461 411
- US-A1- 2016 287 081
- US-A1- 2018 212 112

## Description

### Technical Field

The present invention relates to a light emitting device, and a medical system, an electronic apparatus, and an inspection method using the light emitting device.

### Background

A method of observing lesions, called a fluorescence imaging method, has been attracting attention recently in the medical field. The fluorescence imaging method is a method of observing a lesion by administering a fluorescent drug that selectively binds to the lesion such as a tumor to a subject, exciting the fluorescent drug with a specific light, and detecting and imaging fluorescence emitted from the fluorescent drug by an image sensor. The fluorescence imaging method makes it possible to observe lesions that are difficult to observe visually.

As a typical fluorescence imaging method, a fluorescence imaging method (ICG fluorescence method) using indocyanine green (ICG) as a fluorescent drug is known. The ICG is excited by a near-infrared light (for example, fluorescence peak wavelength is 770 nm), which easily penetrates a living body, and emits a near-infrared light of a longer wavelength (for example, fluorescence peak wavelength is 810 nm). Therefore, by detecting the fluorescence emitted from the ICG, observation of a lesion inside the living body is possible. The ICG fluorescence method is a minimally invasive medical technology that achieves the observation of lesions inside the living body without damaging the living body.

The fluorescence imaging method, such as the ICG fluorescence method, uses at least a device that emits a near-infrared light. As an optoelectronic element emitting near-infrared fluorescence, Patent Literature 1 discloses an optoelectronic element that includes a semiconductor chip emitting a primary beam and a conversion material including Cr³⁺ ions and/or Ni²⁺ ions.

EP 3 904 488 A1 describes a light-emitting device that includes a light source that radiates primary light; and a first phosphor that absorbs the primary light and converts the primary light into first wavelength-converted light having a longer wavelength than the primary light, wherein the primary light is laser light. The first wavelength-converted light includes fluorescence based on electron energy transition of Cr³⁺, and a fluorescence spectrum of the first wavelength-converted light has a maximum fluorescence intensity value in region of a wavelength exceeding 710 nm.

WO 2018/207703 A1 describes a light-emitting device including a semiconductor light-emitting element that emits ultraviolet light or visible light and a phosphor that absorbs ultraviolet light or visible light emitted from the semiconductor light-emitting element and emits light in the infrared region, wherein an emission peak wavelength in the infrared region of the phosphor emitting in the infrared region is from 750 to 1,050 nm, and the half width of an emission peak waveform is more than 50 nm.

### Citation List

### Patent Literature

PTL1: Japanese Translation of PCT International Application Publication No. JP-T-2018-518046

### Summary

As described above, to utilize a fluorescence imaging method, such as the ICG fluorescence method, at least a device for emitting near-infrared light is used. In contrast, to normally visually observe the state of a mucosal surface layer through an image projected by an image sensor for visible light or through a lens, it is preferable that visible light is also emitted. Therefore, if a device for simultaneously emitting visible light and near-infrared light is provided, it is possible to achieve both normal observation using visible light and special observation using near-infrared light.

However, when mixed light of visible light and near-infrared light is emitted, a light component of the visible light, which is closer to the near-infrared range, is easily detected by the near-infrared light image sensor. Therefore, a light component other than the near-infrared fluorescence emitted from, for example, a fluorescent drug, is also detected by the near-infrared light image sensor to generate noise, and it is difficult to observe the lesion with high contrast.

The present invention has been made in consideration of such an issue as described above. It is an object of the present invention to provide a light emitting device that emits near-infrared light and visible light to obtain a high-contrast observation result, and a medical system, an electronic apparatus, and an inspection method using the light emitting device.

In response to the above issue, a light emitting device according to a first aspect of the present invention includes: a light source configured to emit a primary light; a first wavelength converter including a first phosphor that absorbs the primary light and converts the primary light into a first wavelength-converted light having a wavelength longer than that of the primary light; and a second wavelength converter including a second phosphor that absorbs the primary light and converts the primary light into a second wavelength-converted light having a wavelength longer than that of the primary light. The first wavelength-converted light is a fluorescence having a light component over an entire wavelength range of 700 nm or more to 800 nm or less and having a peak where a fluorescence intensity shows a maximum value in a wavelength range of 700 nm or more, and the second wavelength-converted light is a fluorescence having a peak where a fluorescence intensity shows a maximum value in a wavelength range of 380 nm or more to less than 700 nm. The light emitting device emits a first output light including the first wavelength-converted light and a second output light including the second wavelength-converted light alternately in time.

A medical system according to a second aspect of the present invention includes the light emitting device according to the first aspect.

An electronic apparatus according to a third aspect of the present invention includes the light emitting device according to the first aspect.

An inspection method according to a fourth aspect of the present invention uses the light emitting device according to the first aspect.

### Brief Description of Drawings

[FIG. 1] FIG. 1 is a schematic sectional view of an example of a light emitting device according to a first embodiment.
[FIG. 2] FIG. 2 is a schematic sectional view of an example of a light emitting device according to a second embodiment.
[FIG. 3] FIG. 3 is a schematic plan view of an example of a chopper.
[FIG. 4] FIG. 4 is a schematic sectional view of an example of a light emitting device according to a third embodiment.
[FIG. 5] FIG. 5 is a schematic sectional view of an example of a light emitting device according to a fourth embodiment.
[FIG. 6] FIG. 6 is a schematic plan view of an example of a phosphor wheel.
[FIG. 7] FIG. 7 is a schematic sectional view of an example of a light emitting device according to a fifth embodiment.
[FIG. 8] FIG. 8 is a schematic sectional view of an example of a light emitting device according to a sixth embodiment.
[FIG. 9] FIG. 9 is a schematic sectional view of an example of a light emitting device according to a seventh embodiment.
[FIG. 10] FIG. 10 is a schematic diagram illustrating a configuration of an endoscope according to a present embodiment.
[FIG. 11] FIG. 11 is a schematic diagram illustrating a configuration of an endoscope system according to the present embodiment.
[FIG. 12] FIG. 12 is a fluorescence spectrum of fluorescence emitted by a first phosphor according to an example.
[FIG. 13] FIG. 13 is a fluorescence spectrum of fluorescence emitted by a second phosphor according to the example.

### Description of Embodiments

A detailed description is given below of a light emitting device, and a medical system, an electronic apparatus, and an inspection method using the light emitting device according to a present embodiment. Note that dimensional ratios in the drawings are exaggerated for convenience of explanation, and are sometimes different from actual ratios.

### [Light emitting device]

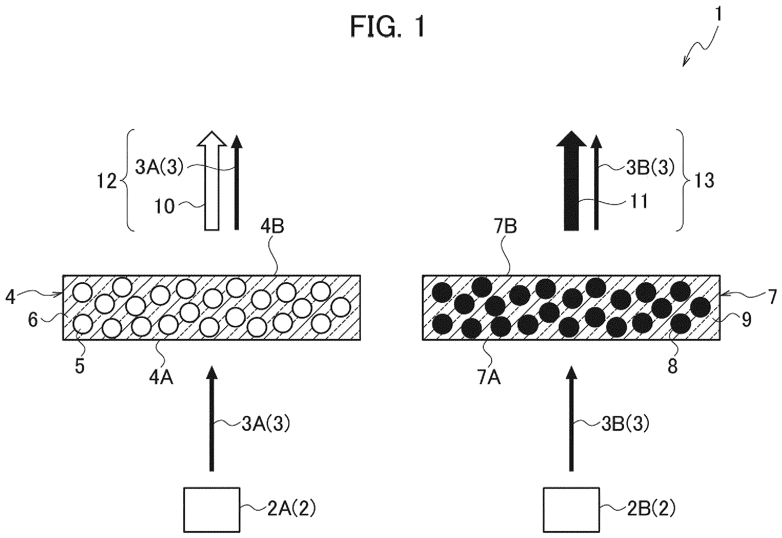

A light emitting device according to the present embodiment is described below with reference to FIGS. 1 to 9.

### [First embodiment]

As illustrated in FIG. 1, a light emitting device 1 according to the present embodiment includes a light source 2, a first wavelength converter 4, and a second wavelength converter 7. The light source 2 emits a primary light 3. The first wavelength converter 4 includes a first phosphor 5. The first phosphor 5 absorbs the primary light 3 and converts it into a first wavelength-converted light 10 having a wavelength longer than that of the primary light 3. The second wavelength converter 7 includes a second phosphor 8. The second phosphor 8 absorbs the primary light 3 and converts it into a second wavelength-converted light 11 having a wavelength longer than that of the primary light 3.

The first wavelength-converted light 10 is a fluorescence having a light component over the entire wavelength range of 700 nm or more to 800 nm or less and having a peak where the fluorescence intensity shows a maximum value in a wavelength range of 700 nm or more. The second wavelength-converted light 11 is a fluorescence having a peak where the fluorescence intensity shows a maximum value in a wavelength range of 380 nm or more to less than 700 nm. The light emitting device 1 emits a first output light 12 including the first wavelength-converted light 10 and a second output light 13 including the second wavelength-converted light 11 alternately in time.

That is, when the primary light 3 emitted by the light source 2 enters the first wavelength converter 4, the first wavelength converter 4 emits the first wavelength-converted light 10 mainly including a near-infrared fluorescent component. In contrast, when the primary light 3 emitted by the light source 2 enters the second wavelength converter 7, the second wavelength converter 7 emits the second wavelength-converted light 11 mainly including a visible light component.

The near-infrared fluorescent component is excellent in living body permeability, has a wide fluorescence spectrum width, and excites, for example, a fluorescent drug used in a fluorescence imaging method, so that the fluorescent drug emits a near-infrared fluorescence having a wavelength longer than that of the exciting near infrared. The near-infrared fluorescence emitted by the fluorescent drug is detected and imaged by a near-infrared light image sensor, and thus special observation is possible. In contrast, the visible fluorescent component emitted by the light emitting device 1 is highly visible and useful for normal visual observation of a diseased part of a living body.

The light emitting device 1 emits the first output light 12 including the first wavelength-converted light 10 and the second output light 13 including the second wavelength-converted light 11 alternately in time. Thus, the near-infrared fluorescence image sensor detects the near-infrared fluorescence component having a relatively high intensity ratio while the intensity of the noise component is relatively low. Therefore, the light emitting device 1 according to the present embodiment emits near-infrared light and visible light to obtain a high-contrast observation result by utilizing the time difference.

The light source 2 may be a single light source or may include at least two light sources as illustrated in FIG. 1. At least two light sources 2 may be the same in terms of the color tone and the output characteristics of the primary light 3 or may be different in accordance with the excitation characteristics of the phosphor. Similarly, the primary light 3 emitted by each light source 2 may have the same light component or different light components.

In the present embodiment, as illustrated in FIG. 1, the light source 2 includes a first light source 2A, and a second light source 2B. The first light source 2A emits a primary light 3A, and the second light source 2B emits a primary light 3B. The optical axis of the primary light 3A is oriented perpendicular to a front 4A of the first wavelength converter 4, and the optical axis of the primary light 3B is oriented perpendicular to a front 7A of the second wavelength converter 7. That is, in the present embodiment, the optical axes of the primary light 3A and the primary light 3B have the same orientation. The primary light 3A is emitted to the first wavelength converter 4 to excite the first phosphor 5 included in the first wavelength converter 4. The primary light 3B is emitted to the second wavelength converter 7 to excite the second phosphor 8 included in the second wavelength converter 7.

As illustrated in FIG. 1, the primary light 3A for exciting the first phosphor 5 and the primary light 3B for exciting the second phosphor 8 may have different optical axes. This enables the primary light 3A and the primary light 3B to be separately controlled by the first light source 2A and the second light source 2B, thereby facilitating ON-OFF control of the first wavelength-converted light 10 and the second wavelength-converted light 11.

In the present embodiment, the primary light 3A and the primary light 3B are emitted alternately in time. That is, while the first light source 2A is turned on, the second light source 2B is turned off, and while the first light source 2A is turned off, the second light source 2B is turned on. Therefore, the light emitting device 1 emits the first output light 12 and the second output light 13 alternately in time.

Preferably, the primary light 3 is a laser light. The laser light is a high-output point light source with strong directivity, and thus it not only reduces the size of the optical system and the diameter of the light guiding part but also improves the coupling efficiency of the laser light to an optical fiber. Accordingly, the light emitting device 1 that facilitates high output is obtained. Preferably, the laser light is emitted by a semiconductor light emitting device from the viewpoint of miniaturization of the light emitting device 1.

Preferably, the spectrum of the light emitted by the light source 2 has a peak where the intensity shows a maximum value in a range of 400 nm or more to less than 500 nm. Also preferably, the spectrum of the light emitted by the light source 2 has a peak where the intensity shows a maximum value in a wavelength range of 420 nm or more to less than 480 nm, and the light emitted by the light source 2 is blue light. The spectrum of the light emitted by the light source 2 has a peak where the intensity shows a maximum value more preferably in a wavelength range of 430 nm or more to less than 480 nm, even more preferably in a wavelength range of 440 nm or more to less than 470 nm. Thus, the first phosphor 5 and the second phosphor 8 are excited with high efficiency, which enables the light emitting device 1 to emit high-output near-infrared light.

The light source 2 may include a red laser device. The light source 2 may include a blue laser device. Preferably, the red laser device is included in the first light source 2A that excites the first phosphor 5. The red laser device has a small energy difference from the near-infrared light component and a small energy loss associated with wavelength conversion, which is preferable in achieving high efficiency of the light emitting device 1. Preferably, the red laser device has a peak where the fluorescence intensity shows a maximum value in a wavelength range of 600 nm or more to less than 660 nm, particularly 610 nm or more to less than 660 nm. In contrast, a laser device with high efficiency and high output is easily available for the blue laser device, and thus the blue laser device is preferable in achieving high output of the light emitting device 1. Preferably, the light source 2 includes a blue laser device as an excitation source and emits blue laser light. Thus, the first phosphor 5 and the second phosphor 8 are excited with high efficiency and high output, which enables the light emitting device 1 to emit high-output near-infrared light.

Preferably, the light source 2 includes a solid-state light emitting device, and the above-described blue light is emitted by the solid-state light emitting device. In this way, a small-sized light emitting device with high reliability is used as a light emitting source of the above-described blue light, which provides a small-sized light emitting device 1 with high reliability.

The solid-state light emitting device is a light emitting device that emits the primary light 3. Any solid-state light emitting device is usable as long as it emits the primary light 3 with a high energy density. The solid-state light emitting device is preferably at least one of a laser device or a light-emitting diode (LED), more preferably a laser device. The light source 2 may be, for example, a surface emitting laser diode.

The rated light output of the solid-state light emitting device is preferably 1 W or more, more preferably 3 W or more. This enables the light source 2 to emit the primary light 3 with high output, and thus the light emitting device 1 that facilitates high output is obtained.

The upper limit of the rated light output is not limited, and the rated light output is increased by the light source 2 having a plurality of solid-state light emitting devices. However, for practical purposes, the rated light output is preferably less than 10 kW, more preferably less than 3 kW.

The light density of the primary light 3 is preferably more than 0.5 W/mm², more preferably more than 3 W/mm², still more preferably more than 10 W/mm². The light density may exceed 30 W/mm². In this way, the first phosphor 5 and the second phosphor 8 are photoexcited at high density, which enables the light emitting device 1 to emit a fluorescent component of high output.

Preferably, the correlated color temperature of a mixed light of the primary light 3 and the second wavelength-converted light 11 is 2500 K or more and less than 7000 K. The correlated color temperature is more preferably 2700 K or more and less than 5500 K, more preferably 2800 K or more and less than 3200 K or 4500 K or more and less than 5500 K. The output light with a correlated color temperature within the above-described range is a white output light, and a diseased part visible through an image display device or an optical device appears similar to the diseased part observed under natural light. Therefore, the light emitting device 1 is obtained that easily makes use of the medical experience of doctors, which is preferable for medical use.

Preferably, the second wavelength-converted light 11 has a light component over the entire wavelength range of 500 nm or more to less than 580 nm. Since the second wavelength-converted light 11 has such a light component, the light emitting device 1 effectively emits a fluorescent component advantageous for visual inspection. The second wavelength-converted light 11 may have a light component over the entire wavelength range of 500 nm or more to less than 600 nm.

The first wavelength-converted light 10 has a light component over the entire wavelength range of 700 nm or more to 800 nm or less. More preferably, the first wavelength-converted light 10 has a light component over the entire wavelength range of 750 nm or more to 800 nm or less. This enables the light emitting device 1 to emit a near-infrared excitation light that efficiently excites a drug, even when the drug has a near-infrared light absorption property that is likely to vary. Thus, the light emitting device 1 increases the amount of near-infrared light emitted by a fluorescent drug, or heat rays emitted by a photosensitive drug or active oxygen generated upon excitation of the photosensitive drug.

The first phosphor 5 is preferably activated with a transition metal ion, more preferably activated with Cr³⁺. This makes it easier to obtain a fluorescence with a broad fluorescence spectrum width and a light component over the entire wavelength range of 700 nm or more to 800 nm or less, as the first wavelength-converted light 10.

The first wavelength-converted light 10 is a fluorescence having a peak where the fluorescence intensity shows a maximum value in a wavelength range of 700 nm or more. Preferably, the first wavelength-converted light 10 has a peak where the fluorescence intensity shows a maximum value in a wavelength range of 710 nm or more. This enables the light emitting device 1 to emit a fluorescence including a large number of near-infrared light components with high living body permeability.

Preferably, the first wavelength-converted light 10 includes a fluorescence based on the electronic energy transition of Cr³⁺. Preferably, the fluorescence spectrum of the first wavelength-converted light 10 has a peak where the fluorescence intensity shows a maximum value in a wavelength range exceeding 720 nm. This enables the first phosphor 5 to emit a fluorescence in which a broad spectral component of a short afterglow property is more dominant than a linear spectral component of a long afterglow property. As a result, the light emitting device 1 emits a light including a large amount of the near-infrared component. The linear spectrum component is a fluorescence component based on the electron energy transition (spin-forbidden transition) of ²E→⁴A₂(t₂³) of Cr³⁺ and has a peak where the fluorescence intensity shows a maximum value in a wavelength range of 680 nm to 720 nm. The broad spectral component is a fluorescence component based on the electron energy transition (spin-allowed transition) of ⁴T₂(t₂²e)→⁴A₂(t₂³) of Cr³⁺ and has a peak where the fluorescence intensity shows a maximum value in a wavelength range exceeding 720 nm.

The fluorescence spectrum of the first wavelength-converted light 10 may have a peak where the fluorescence intensity shows a maximum value in a wavelength range of more than 720 nm to 900 nm or less. The fluorescence spectrum of the first wavelength-converted light 10 more preferably has a peak where the fluorescence intensity shows a maximum value in a wavelength range exceeding 730 nm, further more preferably has a peak where the fluorescence intensity shows a maximum value in a wavelength range exceeding 750 nm.

The 1/10 afterglow time of the first wavelength-converted light 10 is preferably less than 1 ms, more preferably less than 300 µs, still more preferably less than 100 µs where the afterglow time is a short afterglow property. Thus, even when the light density of the excitation light for exciting the first phosphor 5 is high, the output of the first wavelength-converted light 10 hardly saturates. Thus, the light emitting device 1 capable of emitting a high output near-infrared light is obtained. Note that the 1/10 afterglow time means time τ_{1/10} taken from the time when the maximum light emission intensity is shown to the time when the intensity becomes 1/10 of the maximum light emission intensity.

Preferably, the 1/10 afterglow time of the first wavelength-converted light 10 is longer than the 1/10 afterglow time of the second wavelength-converted light 11. Preferably, the 1/10 afterglow time of the first wavelength-converted light 10 is specifically 10 µs or more. Note that the 1/10 afterglow time of the first wavelength-converted light 10 activated with Cr³⁺ is longer than the 1/10 afterglow time of a short afterglow (less than 10 µs) fluorescence based on a parity-allowed transition of Ce³⁺, Eu²⁺, or the like. This is because the first wavelength-converted light 10 is a fluorescence based on the electron energy transition of the spin-allowed type of Cr³⁺, which has relatively long afterglow time.

Preferably, in the fluorescence spectrum of the first wavelength-converted light 10, the spectral width at an intensity of 80% of the maximum value of the fluorescence intensity is 20 nm or more and less than 80 nm. Thus, the main component of the first wavelength-converted light 10 becomes a broad spectrum component. Therefore, even when there is a variation in the wavelength dependence of the sensitivity of a fluorescent drug or photosensitive drug in a medical field using a fluorescence imaging method or photodynamic therapy (PDT method), the light emitting device 1 emits high output near-infrared light that enables these drugs to function sufficiently.

Preferably, in the fluorescence spectrum of the first wavelength-converted light 10, the ratio of the fluorescence intensity at a wavelength of 780 nm to the maximum fluorescence intensity exceeds 30%. The ratio of the fluorescence intensity at a wavelength of 780 nm to the maximum fluorescence intensity more preferably exceeds 60%, even more preferably exceeds 80%. This enables the first phosphor 5 to emit a fluorescence including a large amount of a fluorescent component of a near-infrared wavelength range (650 to 1000 nm) through which light easily penetrates the living body, which is called a "living body window". Therefore, the above-described light emitting device 1 increases the light intensity of the near infrared that penetrates the living body.

Preferably, the fluorescence spectrum of the first wavelength-converted light 10 does not leave a trail of a linear spectral component derived from the electronic energy transition of Cr³⁺. That is, preferably, the first wavelength-converted light 10 has only a broad spectral component (short afterglow property) having a peak where the fluorescence intensity shows a maximum value in a wavelength range exceeding 720 nm. Thus, the first phosphor 5 does not include a long afterglow fluorescent component due to the spin-forbidden transition of Cr³⁺ but only includes a short afterglow fluorescent component due to the spin-allowed transition of Cr³⁺. Thus, even when the light density of the excitation light for exciting the first phosphor 5 is high, the output of the first wavelength-converted light 10 hardly saturates. Therefore, the light emitting device 1 of a point light source capable of emitting a near-infrared light of higher output is also obtained.

Preferably, the first phosphor 5 includes no activator other than Cr³⁺. This enables the light absorbed by the first phosphor 5 to be converted into only the fluorescence based on the electronic energy transition of Cr³⁺, which provides the light emitting device 1 with easy design of output light for maximizing the output ratio of the near-infrared fluorescent component.

Preferably, the first phosphor 5 includes two or more kinds of Cr³⁺-activated phosphors. This enables the output light component in at least the near-infrared wavelength range to be controlled, which provides the light emitting device 1 with easy adjustment of the spectral distribution in accordance with the application utilizing the near-infrared fluorescence component.

The first phosphor 5 is preferably an oxide-based phosphor, more preferably an oxide phosphor. The oxide-based phosphor means a phosphor including oxygen but not nitrogen or sulfur. The oxide-based phosphor may include at least one selected from the group consisting of an oxide, a complex oxide, and a compound including oxygen or halogen as an anion.

Since the oxide is stable in the atmosphere, even when the oxide phosphor generates heat due to high density photoexcitation by laser light, it is difficult for phosphor crystals to be altered by oxidation in the atmosphere, as occurs in nitride phosphors. Therefore, when all the phosphors in the first wavelength converter 4 are oxide phosphors, the light emitting device 1 that is highly reliable is obtained.

Preferably, the first phosphor 5 has a garnet crystal structure. Preferably, the first phosphor 5 is an oxide phosphor with a garnet crystal structure. Since a garnet phosphor is easily deformed in composition and provides a number of phosphor compounds, a crystal field around Cr³⁺ is easily adjusted, and the color tone of fluorescence based on the electronic energy transition of Cr³⁺ is easily controlled.

The phosphor with a garnet structure, especially the oxide, has a polyhedral particle shape close to a sphere and has excellent dispersibility of a phosphor particle group. Therefore, when the phosphor included in the first wavelength converter 4 has a garnet structure, the first wavelength converter 4 excellent in light transmittance is manufactured relatively easily, which enables higher output of the light emitting device 1. Further, since a phosphor with a garnet crystal structure has practical experience as a phosphor for LEDs, the light emitting device 1 that is a highly reliable is obtained when the first phosphor 5 has the garnet crystal structure.

The first phosphor 5 may include at least one phosphor selected from the group consisting of: Lu₂CaMg₂(SiO₄)₃:Cr³⁺, Y₃Ga₂(AlO₄)₃:Cr³⁺, Y₃Gaz(GaO₄)₃:Cr³⁺, Gd₃Ga₂(AlO₄)₃:Cr³⁺, Gd₃Ga₂(GaO₄)₃:Cr³⁺, (Y,La)₃Ga₂(GaO₄)₃:Cr³⁺, (Gd,La)₃Ga₂(GaO₄)₃:Cr³⁺, Ca₂LuZr₂(AlO₄)₃:Cr³⁺, Ca₂GdZr₂(AlO₄)₃:Cr³⁺, Lu₃Sc₂(GaO₄) ₃:Cr³⁺, Y₃Sc₂(AlO₄)₃: Cr³⁺, Y₃Sc₂(GaO₄)₃:Cr³⁺, Gd₃Sc₂(GaO₄)₃:Cr³⁺, La₃Sc₂(GaO₄)₃:Cr³⁺, Ca₃Sc₂(SiO₄)₃:Cr³⁺, Ca₃Sc₂(GeO₄)₃:Cr³⁺, BeAl₂O₄:Cr³⁺, LiAl₅O₈:Cr³⁺, LiGa₅O₈:Cr³⁺, Mg₂SiO₄:Cr³⁺, Li⁺, La₃Ga₅GeO₁₄:Cr³⁺, and La₃Ga_{5.5}Nb _{0.5}O₁₄:Cr³⁺.

As described above, the first wavelength-converted light 10 has a near-infrared fluorescence component. This enables the light emitting device 1 to efficiently excite a fluorescent drug, such as ICG, or a photosensitive drug (which is also a fluorescent drug), such as phthalocyanine.

Preferably, the second phosphor 8 is activated with at least one of Ce³⁺ or Eu²⁺. This makes it easy to obtain the second wavelength-converted light 11 with a peak where the fluorescence intensity shows a maximum value in a wavelength range of 380 nm or more to less than 700 nm. Preferably, the second phosphor 8 is activated with Ce³⁺.

The second phosphor 8 may be at least one of an oxide-based phosphor, such as an oxide or a halogen oxide, or a nitride-based phosphor, such as a nitride or an oxynitride.

Preferably, the second phosphor 8 is a Ce³⁺-activated phosphor having a matrix of a compound with at least one, as a main component, selected from the compound group consisting of a garnet type crystal structure, a calcium ferrite type crystal structure, and a lanthanum silicon nitride (La₃Si₆N₁₁) type crystal structure. Preferably, the second phosphor 8 is a Ce³⁺-activated phosphor having a matrix of at least one selected from the compound group consisting of a garnet type crystal structure, a calcium ferrite type crystal structure, and a lanthanum silicon nitride (La₃Si₆N₁₁) type crystal structure. Using the above-described second phosphor 8 provides output light with a large amount of a light component from green to yellow.

Preferably, the second phosphor 8 is specifically a Ce³⁺-activated phosphor having a matrix of a compound with at least one, as a main component, selected from the group consisting of M₃RE₂(SiO₄)₃, RE₃Al₂(AlO₄)₃, MRE₂O₄, and RE₃Si₆N₁₁. Preferably, the second phosphor 8 is a Ce³⁺-activated phosphor having a matrix of at least one selected from the group consisting of M₃RE₂(SiO₄)₃, RE₃Al₂(AlO₄)₃, MRE₂O₄, and RE₃Si₆N₁₁. Preferably, the second phosphor 8 is a Ce³⁺-activated phosphor having a matrix of a solid solution having the above-described compound as an end component. Note that M is an alkaline earth metal, and RE is a rare earth element.

The above-described second phosphor 8 well absorbs light in a wavelength range of 430 nm or more to 480 nm or less and converts it to green to yellow light with a peak where the fluorescence intensity shows a maximum value in a wavelength range of 540 nm or more to less than 590 nm with high efficiency. Therefore, a visible light component is easily obtained by using such a phosphor as the second phosphor 8 with the light source 2 that emits cold color light in a wavelength range of 430 nm or more to 480 nm or less as the primary light 3.

At least the second wavelength converter 7 may further include a third phosphor that absorbs the primary light 3 and emits a third wavelength-converted light. Preferably, the third wavelength-converted light is included in the second output light 13, and the second output light 13 is a white output light. For example, when the primary light 3 is a blue light, such a white light is emitted by the light emitting device 1 by additive color mixing with this blue light.

Preferably, the first wavelength converter 4 includes an inorganic material. Here, the inorganic material means materials other than organic materials and includes ceramics and metals as a concept. When the first wavelength converter 4 includes an inorganic material, the first wavelength converter 4 has the heat conductivity higher than that of the wavelength converter including an organic material, such as a sealing resin, thereby facilitating the heat radiation design. Thus, the temperature rise of the first wavelength converter 4 is effectively prevented even when the phosphor is photoexcited with high density by the primary light 3 emitted from the light source 2. As a result, the temperature quenching of the phosphor in the first wavelength converter 4 is prevented, and thus higher output of light emission is possible. Accordingly, the heat dissipation of the first phosphor 5 is improved, and thus the decrease in output of the phosphor due to temperature quenching is prevented, and high output near-infrared light is emitted. For the same reason as for the first wavelength converter 4, preferably, the second wavelength converter 7 also includes an inorganic material.

Preferably, at least one of all of the first wavelength converter 4 or all of the second wavelength converter 7 is made of an inorganic material. As a result, the heat dissipation of the first phosphor 5 and/or the second phosphor 8 is improved, and thus the decrease in output of the phosphor due to temperature quenching is prevented, and the light emitting device 1 that emits a high output near-infrared fluorescence and/or visible light is obtained. Preferably, all of the first wavelength converter 4 and all of the second wavelength converter 7 are made of inorganic material.

At least one of the first phosphor 5 or the second phosphor 8 may be a ceramic. This increases the thermal conductivity of at least one of the first wavelength converter 4 or the second wavelength converter 7, which provides the light emitting device 1 with less heat generation and high output. Here, the ceramic means a sintered body in which particles are bonded to each other.

As illustrated in FIG. 1, preferably, the first wavelength converter 4 further includes a first sealing material 6 that disperses the first phosphor 5, in addition to the first phosphor 5. Preferably, the first wavelength converter 4 has the first phosphor 5 dispersed in the first sealing material 6. By dispersing the first phosphor 5 in the first sealing material 6, the light emitted to the first wavelength converter 4 is efficiently absorbed and wavelength-converted into a near-infrared light. Further, the first wavelength converter 4 is easily formed into a sheet shape or a film shape. For the same reason as for the first wavelength converter 4, preferably, a second sealing material 9 that disperses the second phosphor 8 is further included in addition to the second phosphor 8.

Preferably, the first sealing material 6 is at least one of an organic material or an inorganic material, particularly at least one of a transparent (light transmitting) organic material or a transparent (light transmitting) inorganic material. Examples of the sealing material of the organic material include a transparent organic material, such as a silicone resin. Examples of the sealing material of the inorganic material include a transparent inorganic material, such as a low melting point glass. Preferably, the second sealing material 9 is the same as the first sealing material 6.

As described above, the first wavelength converter 4 preferably includes an inorganic material, and thus the first sealing material 6 preferably includes an inorganic material. Preferably, zinc oxide (ZnO) is used as the inorganic material. This further enhances the heat dissipation of the phosphor, which prevents the output of the phosphor from decreasing due to temperature quenching and provides the light emitting device 1 that emits high output near-infrared light. Preferably, the second sealing material 9 is the same as the first sealing material 6.

The first wavelength converter 4 may not use the first sealing material 6, and the second wavelength converter 7 may not use the second sealing material 9. In this case, the phosphor may be fixed to each other by using an organic or inorganic binder. The phosphor is fixable to each other by using the heating reaction of the phosphor. As the binder, a commonly used resin-based adhesive, ceramic fine particles, low melting point glass, or the like is usable. The first wavelength converter 4 that does not use the first sealing material 6, or the second wavelength converter 7 that does not use the second sealing material 9 is made thin, and thus it is suitably used for the light emitting device 1.

Next, the operation of the light emitting device 1 according to the present embodiment is described. As illustrated in FIG. 1, in the light emitting device 1 according to the present embodiment, first, the first wavelength converter 4 and the second wavelength converter 7 are respectively irradiated with the primary light 3A emitted by the first light source 2A and the primary light 3B emitted by the second light source 2B alternately in time. The primary light 3A is emitted to the front 4A of the first wavelength converter 4, and the emitted primary light 3A passes through the first wavelength converter 4. The primary light 3B is emitted to the front 7A of the second wavelength converter 7, and the emitted primary light 3B passes through the second wavelength converter 7. Then, when the primary light 3A passes through the first wavelength converter 4, the first phosphor 5 included in the first wavelength converter 4 absorbs a part of the primary light 3A to emit the first wavelength-converted light 10. Similarly, when the primary light 3B passes through the second wavelength converter 7, the second phosphor 8 included in the second wavelength converter 7 absorbs a part of the primary light 3B to emit the second wavelength-converted light 11. In this way, the first output light 12 including the primary light 3A and the first wavelength-converted light 10 is emitted from the back 4B of the first wavelength converter 4. Similarly, the second output light 13 including the primary light 3B and the second wavelength-converted light 11 is emitted from the back 7B of the second wavelength converter 7.

In this way, the first light source 2A and the second light source 2B repeatedly turn on and off alternately in time, which enables the light emitting device 1 to emit the first output light 12 and the second output light 13 alternately in time.

### [Second embodiment]

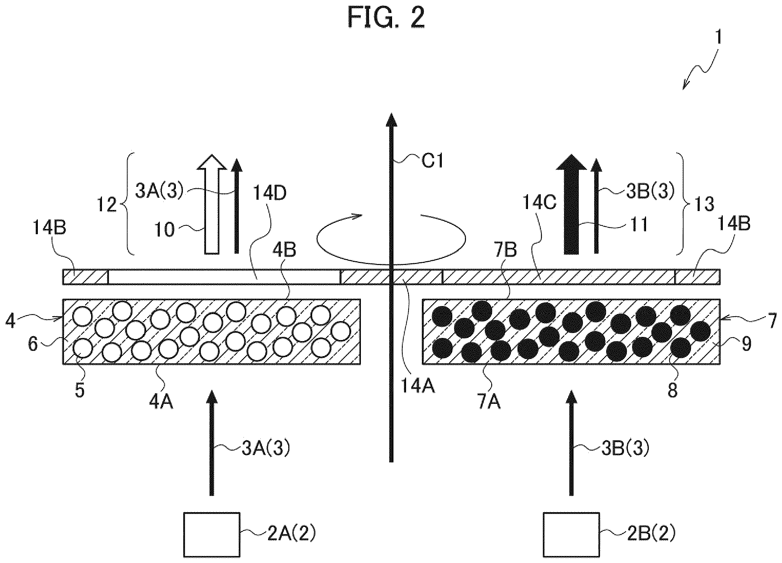

Next, a light emitting device 1 according to a second embodiment is described with reference to FIG. 2. The same components as those of the above-described embodiment are denoted by the same reference numerals, and redundant descriptions are omitted.

As illustrated in FIG. 2, the present embodiment differs from the first embodiment in that a first output light 12 and a second output light 13 are emitted alternately in time by a chopper 14 instead of turning on and off the light source 2.

In the present embodiment, as in the first embodiment, a light source 2 includes a first light source 2A and a second light source 2B. The first light source 2A emits a primary light 3A, and the second light source 2B emits a primary light 3B. The primary light 3A for exciting a first phosphor 5 and the primary light 3B for exciting a second phosphor 8 have different optical axes. In the present embodiment, the primary light 3A and the primary light 3B are each consecutively and continuously emitted. That is, the primary light 3A and the primary light 3B are simultaneously emitted, and while the first light source 2A is on, the second light source 2B is also on.

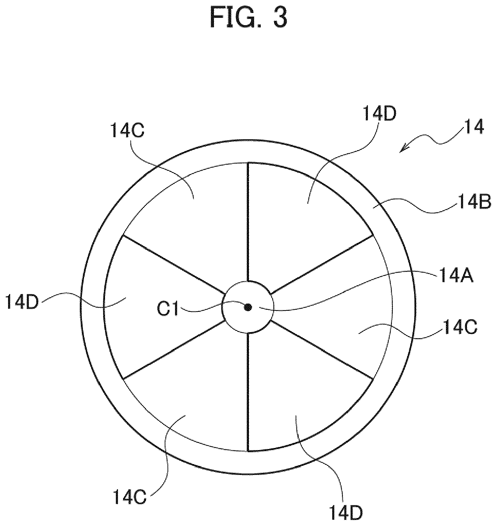

The light emitting device 1 according to the present embodiment includes the chopper 14. The chopper 14 is disposed at a back 4B side of a first wavelength converter 4 and at a back 7B side of a second wavelength converter 7. That is, the first wavelength converter 4 is disposed between the chopper 14 and the first light source 2A, and the second wavelength converter 7 is disposed between the chopper 14 and the second light source 2B. As illustrated in FIG. 3, the chopper 14 is a disk having a central point and is provided to rotate about a rotation axis C1 passing through the central point.

The chopper 14 includes a central portion 14A provided at the center of the disk, an outer frame portion 14B at the periphery of the disk, and shielding and non-shielding portions 14C and 14D between the central portion 14A and the outer frame portion 14B. First wavelength converters 4 and second wavelength converters 7 are alternately disposed in the circumferential direction. The chopper 14 has the shielding portion 14C provided on one side of the rotation axis C1, and the non-shielding portion 14D provided on the opposite side of the rotation axis C1 in a cross section through which the rotation axis C1 passes. The shielding portion 14C is formed of a plate-like member and is formed to shield the first output light 12 and the second output light 13. The non-shielding portion 14D is a space surrounded by the central portion 14A, the outer frame portion 14B, and the shielding portion 14C and is formed to allow the first output light 12 and the second output light 13 to pass through. By rotating the chopper 14 about the rotation axis C1, the first output light 12 and the second output light 13 are shielded by the shielding portions 14C or passed through the non-shielding portions 14D. The chopper 14 is formed so that the second output light 13 passes through the non-shielding portions 14D while the first output light 12 is shielded by the shielding portions 14C, and the first output light 12 passes through the non-shielding portions 14D while the second output light 13 is shielded by the shielding portions 14C. The central portion 14A, the outer frame portion 14B, and the shielding portions 14 C may be integrally formed from the same material.

Next, the operation of the light emitting device 1 according to the present embodiment is described. As illustrated in FIG. 2, in the light emitting device 1 according to the present embodiment, first, the first wavelength converter 4 and the second wavelength converter 7 are simultaneously irradiated with the primary light 3A emitted by the first light source 2A and the primary light 3B emitted by the second light source 2B, respectively. The primary light 3A is emitted to the front 4A of the first wavelength converter 4, and the emitted primary light 3A passes through the first wavelength converter 4. The primary light 3B is emitted to the front 7A of the second wavelength converter 7, and the emitted primary light 3B passes through the second wavelength converter 7. Then, when the primary light 3A passes through the first wavelength converter 4, the first phosphor 5 included in the first wavelength converter 4 absorbs a part of the primary light 3A to emit a first wavelength-converted light 10. Similarly, when the primary light 3B passes through the second wavelength converter 7, the second phosphor 8 included in the second wavelength converter 7 absorbs a part of the primary light 3B to emit a second wavelength-converted light 11. In this way, the first output light 12 including the primary light 3A and the first wavelength-converted light 10 is emitted from a back 4B of the first wavelength converter 4. Similarly, the second output light 13 including the primary light 3B and the second wavelength-converted light 11 is emitted from a back 7B of the second wavelength converter 7.

Then, as the chopper 14 rotates, the first output light 12 emitted from the first wavelength converter 4 repeats being shielded by the shielding portion 14C and passing through the non-shielding portion 14D alternately in time. Similarly, the second output light 13 emitted from the second wavelength converter 7 repeats being shielded by the shielding portions 14C and passing through the non-shielding portions 14D alternately in time. While the first output light 12 is shielded by the shielding portions 14C, the second output light 13 passes through the non-shielding portions 14D, and while the second output light 13 is shielded by the shielding portions 14C, the first output light 12 passes through the non-shielding portions 14D. In this way, the first output light 12 and the second output light 13 are output alternately in time by the chopper 14, and thus the light emitting device 1 emits the first output light 12 and the second output light 13 alternately in time.

### [Third embodiment]

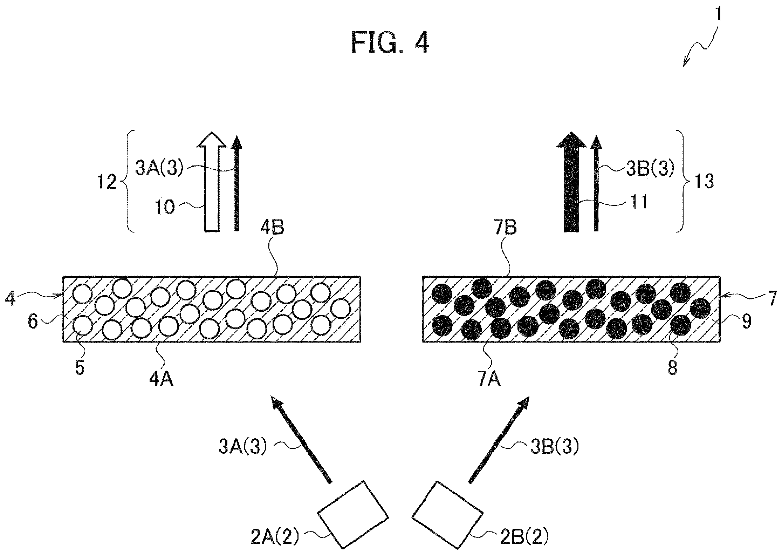

Next, a light emitting device 1 according to a third embodiment is described with reference to FIG. 4. The same components as those of the above-described embodiments are denoted by the same reference numerals, and redundant descriptions are omitted.

As illustrated in FIG. 4, the present embodiment differs from the first embodiment in that the optical axes of a primary light 3A and a primary light 3B are oriented differently from each other.

A first light source 2A is disposed closer to a second wavelength converter 7 than to the center of the cross sectional view of a first wavelength converter 4. A second light source 2B is disposed closer to the first wavelength converter 4 than to the center of the cross sectional view of the second wavelength converter 7. That is, the relative positions of the first light source 2A and the second light source 2B are disposed to be closer to each other than the relative positions of the center of the cross section of the first wavelength converter 4 and the center of the cross section of the second wavelength converter 7. Therefore, in the light emitting device 1 according to the present embodiment, the relative positions of the first light source 2A and the second light source 2B become closer to each other, which reduces the size of the light sources.

Therefore, the light emitting device 1 according to the present embodiment emits a first output light 12 and a second output light 13 alternately in time, as in the first embodiment.

### [Fourth embodiment]

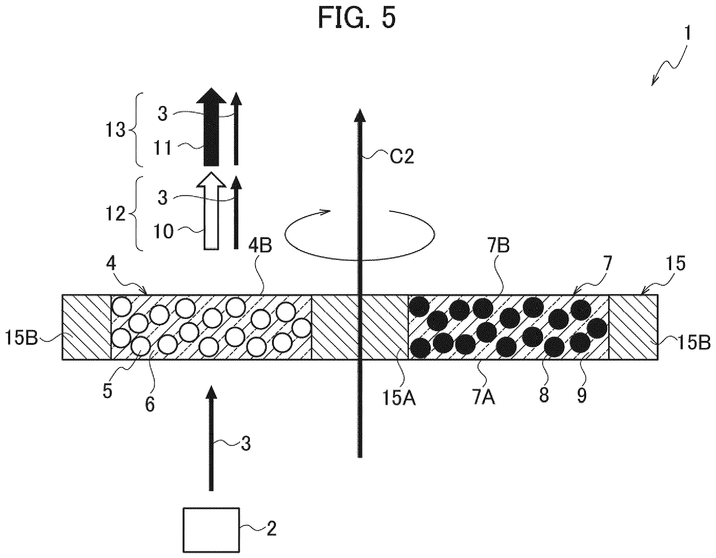

Next, a light emitting device 1 according to a fourth embodiment is described with reference to FIG. 5. The same components as those of the above-described embodiments are denoted by the same reference numerals, and redundant descriptions are omitted.

As illustrated in FIG. 5, the present embodiment differs from the first embodiment in that by changing the positions of a first wavelength converter 4 and a second wavelength converter 7 with respect to a light source 2, a first output light 12 and a second output light 13 are emitted alternately in time.

As in the present embodiment, a primary light 3 for exciting a first phosphor 5 and a primary light 3 for exciting a second phosphor 8 may be emitted by the same light source 2. That is, the light emitting device 1 according to the present embodiment includes a single light source 2. Therefore, the number of light sources 2 is reduced to a necessary minimum, which is advantageous in terms of miniaturization and cost reduction of the light emitting device 1.

As in the present embodiment, the primary light 3 for exciting the first phosphor 5 and the primary light 3 for exciting the second phosphor 8 may have the same optical axis. This minimizes types and the number of light components, which is advantageous in terms of miniaturization and cost reduction of the light emitting device 1.

As in the present embodiment, by changing the positions of the first wavelength converter 4 and the second wavelength converter 7 with respect to the light source 2, the first output light 12 and the second output light 13 may be emitted alternately in time. This enables the primary light 3 for exciting the first phosphor 5 and the primary light 3 for exciting the second phosphor 8 to have the same optical axis. However, as is described later, even if the optical axes are different, by changing the positions of the first wavelength converter 4 and the second wavelength converter 7 with respect to the light source 2, the first output light 12 and the second output light 13 may be emitted alternately in time.

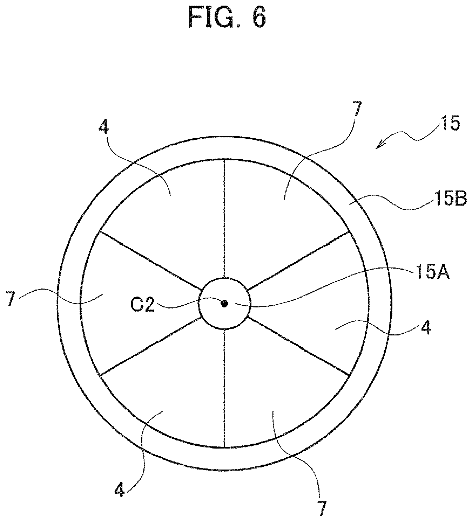

In the present embodiment, the light emitting device 1 includes a phosphor wheel 15 provided with first wavelength converters 4 and second wavelength converters 7. The phosphor wheel 15 is a disk having a central point and is provided to rotate about a rotation axis C2 passing through the central point. As illustrated in FIG. 6, the phosphor wheel 15 includes a central portion 15A at the center of the disk and an outer frame portion 15B at the periphery of the disk. Between the central portion 15A and the outer frame portion 15B, first wavelength converters 4 and second wavelength converters 7 are alternately disposed in the circumferential direction. The phosphor wheel 15 has a first wavelength converter 4 provided on one side of the rotation axis C2, and a second wavelength converter 7 provided on the opposite side of the rotation axis C2 in a cross section through which the rotation axis C2 passes.

The light source 2 is disposed radially outward from the rotation axis C2 and is provided to irradiate either the first wavelength converter 4 or the second wavelength converter 7. The direction of the optical axis of the primary light 3 is perpendicular to a front 4A of the first wavelength converter 4 or a front 7A of the second wavelength converter 7. The phosphor wheel 15 is provided so that the first wavelength converter 4 and the second wavelength converter 7 are irradiated with the primary light 3 alternately in time by rotating about the rotation axis C2. That is, the phosphor wheel 15 is formed so that the second wavelength converter 7 is not irradiated with the primary light 3 while the first wavelength converter 4 is irradiated with the primary light 3, and the first wavelength converter 4 is not irradiated with the primary light 3 while the second wavelength converter 7 is irradiated with the primary light 3. Note that a heat radiating substrate that transmits the primary light 3 may be provided on a light source 2 side of the phosphor wheel 15. This controls the temperature rise of the phosphor wheel 15 caused by the heat generation of the first phosphor 5 having large stokes loss, thus preventing luminous efficiency from decreasing due to temperature quenching of the phosphor and providing the light emitting device 1 that emits high output light. Examples of the material for forming the heat radiating substrate include sapphire.

Next, the operation of the light emitting device 1 according to the present embodiment is described. As illustrated in FIG. 5, in the light emitting device 1 according to the present embodiment, first, the front 4A of the first wavelength converter 4 is irradiated with the primary light 3 emitted by the light source 2. The primary light 3 emitted to the front 4A passes through the first wavelength converter 4. When the primary light 3 passes through the first wavelength converter 4, the first phosphor 5 included in the first wavelength converter 4 absorbs a part of the primary light 3 and emits a first wavelength-converted light 10.

The first wavelength converter 4 and the second wavelength converter 7 change their positions with respect to the light source 2 by the phosphor wheel 15 rotating about the rotation axis C2. Therefore, after the first wavelength converter 4 is irradiates with the primary light 3, the primary light 3 is emitted to the front 7A of the second wavelength converter 7, and the emitted primary light 3 passes through the second wavelength converter 7. Then, when the primary light 3 passes through the second wavelength converter 7, the second phosphor 8 included in the second wavelength converter 7 absorbs a part of the primary light 3 and emits a second wavelength-converted light 11. In this way, the first output light 12 including the primary light 3 and the first wavelength-converted light 10 is emitted from the back 4B of the first wavelength converter 4. Then, the second output light 13 including the primary light 3 and the second wavelength-converted light 11 is emitted from the back 7B of the second wavelength converter 7. As the phosphor wheel 15 rotates, the first wavelength converter 4 and the second wavelength converter 7 are irradiated with the primary light 3 alternately in time, which enables the light emitting device 1 to emit the first output light 12 and the second output light 13 alternately in time.

### [Fifth embodiment]

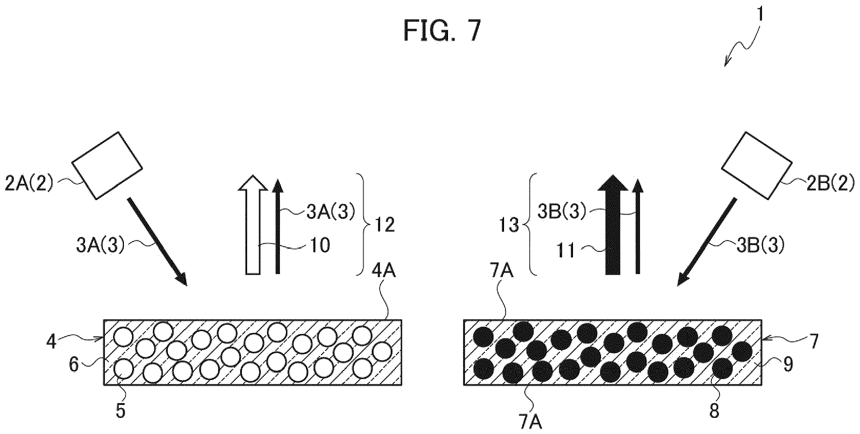

Next, a light emitting device 1 according to a fifth embodiment is described with reference to FIG. 7. The same components as those of the above-described embodiments are denoted by the same reference numerals, and redundant descriptions are omitted.

As illustrated in FIG. 7, in the light emitting device 1 according to the present embodiment, a primary light 3A is emitted to a front 4A of a first wavelength converter 4, and a first wavelength-converted light 10 is emitted from the front 4A of the first wavelength converter 4. A primary light 3B is emitted to a front 7A of a second wavelength converter 7, and a second wavelength-converted light 11 is emitted from the front 7A of the second wavelength converter 7. In these points, the light emitting device 1 according to the present embodiment is different from the light emitting device 1 according to the first embodiment.

In the present embodiment, as illustrated in FIG. 7, a light source 2 includes a first light source 2A and a second light source 2B. The first light source 2A emits the primary light 3A, and the second light source 2B emits the primary light 3B. In the present embodiment, the primary light 3A for exciting a first phosphor 5 and the primary light 3B for exciting a second phosphor 8 have different optical axes, and the primary light 3A and the primary light 3B are emitted alternately in time.

Next, the operation of the light emitting device 1 according to the present embodiment is described. As illustrated in FIG. 7, in the light emitting device 1 according to the present embodiment, first, the first wavelength converter 4 and the second wavelength converter 7 are respectively irradiated with the primary light 3A emitted by the first light source 2A and the primary light 3B emitted by the second light source 2B alternately in time. The primary light 3A is emitted to the front 4A of the first wavelength converter 4, most of the emitted primary light 3A enters the first wavelength converter 4 through the front 4A, and the remaining part is reflected on the surface of the first wavelength converter 4. The primary light 3B is emitted to the front 7A of the second wavelength converter 7, most of the emitted primary light 3B enters the second wavelength converter 7 through the front 7A, and the remaining part is reflected on the surface of the second wavelength converter 7. In the first wavelength converter 4, the first phosphor 5 excited by the primary light 3A emits the first wavelength-converted light 10, and the first wavelength-converted light 10 is emitted from the front 4A. In contrast, in the second wavelength converter 7, the second phosphor 8 excited by the primary light 3B emits the second wavelength-converted light 11, and the second wavelength-converted light 11 is emitted from the front 7A. In this way, the first light source 2A and the second light source 2B repeatedly turn on and off alternately in time, which enables the light emitting device 1 to emit the first output light 12 and the second output light 13 alternately in time.

### [Sixth embodiment]

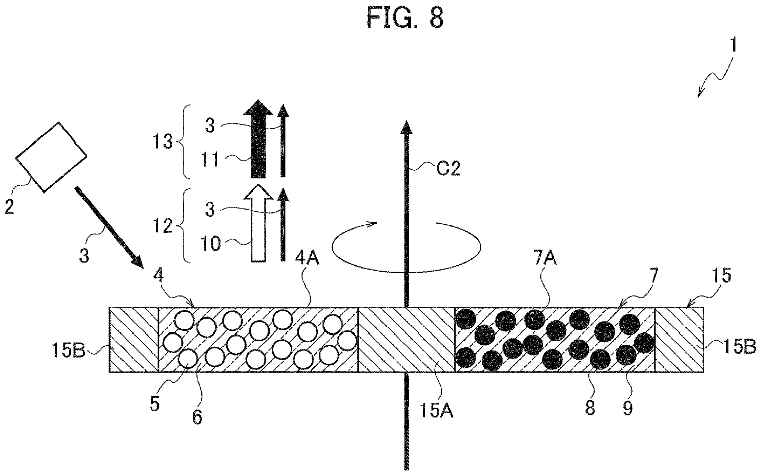

Next, a light emitting device 1 according to a sixth embodiment is described with reference to FIG. 8. The same components as those of the above-described embodiments are denoted by the same reference numerals, and redundant descriptions are omitted.

As illustrated in FIG. 8, in the light emitting device 1 according to the present embodiment, a primary light 3A is emitted to a front 4A of a first wavelength converter 4, and a first wavelength-converted light 10 is emitted from the front 4A of the first wavelength converter 4. A primary light 3B is emitted to a front 7A of a second wavelength converter 7, and a second wavelength-converted light 11 is emitted from the front 7A of the second wavelength converter 7. In these points, the light emitting device 1 according to the present embodiment is different from the light emitting device 1 according to the fourth embodiment.

As illustrated in FIG. 8, the primary light 3 for exciting the first phosphor 5 and the primary light 3 for exciting the second phosphor 8 are emitted by the same light source 2. The primary light 3 for exciting the first phosphor 5 and the primary light 3 for exciting the second phosphor 8 have the same optical axis. By changing the positions of the first wavelength converter 4 and the second wavelength converter 7 with respect to the light source 2, the first output light 12 and the second output light 13 are emitted alternately in time. The light emitting device 1 includes a phosphor wheel 15 provided with first wavelength converters 4 and second wavelength converters 7.

A reflection layer for reflecting the primary light 3, the first wavelength-converted light 10, and the second wavelength-converted light 11 may be provided at the opposite side of the phosphor wheel 15 from the light source 2. The reflective layer is not limited as long as it has a function of reflecting light and includes, for example, a resin including silver and/or titanium oxide. This enables the first wavelength-converted light 10 and the second wavelength-converted light 11 to be taken out from the front side more, which provides the light emitting device 1 that emits high output light. Further, the above-described heat radiation substrate may be provided at the back side of the phosphor wheel 15. This controls the temperature rise of the phosphor wheel 15 caused by the heat generation of the first phosphor 5 having large stokes loss, thus preventing luminous efficiency from decreasing due to temperature quenching of the phosphor and providing the light emitting device 1 that emits high output light.

Next, the operation of the light emitting device 1 according to the present embodiment is described. As illustrated in FIG. 8, in the light emitting device 1 according to the present embodiment, first, the front 4A of the first wavelength converter 4 is irradiated with the primary light 3 emitted by the light source 2. Most of the primary light 3 emitted to the front 4A enters the first wavelength converter 4, and the remaining part is reflected on the surface of the first wavelength converter 4. In the first wavelength converter 4, the first phosphor 5 excited by the primary light 3 emits the first wavelength-converted light 10, and the first wavelength-converted light 10 is emitted from the front 4A.

As the phosphor wheel 15 rotates about the rotation axis C2, the first wavelength converter 4 and the second wavelength converter 7 change their positions with respect to the light source 2. Therefore, after the first wavelength converter 4 is irradiated with the primary light 3, the primary light 3 emitted by the light source 2 is emitted to the front 7A of the second wavelength converter 7. Most of the emitted primary light 3 enters the second wavelength converter 7 through the front 7A, and the remaining part is reflected by the surface of the second wavelength converter 7. In the second wavelength converter 7, the second phosphor 8 excited by the primary light 3 emits the second wavelength-converted light 11, and the second wavelength-converted light 11 is emitted from the front 7A. In this way, as the phosphor wheel 15 rotates, the first wavelength converter 4 and the second wavelength converter 7 emit the primary light 3 alternately in time, which enables the light emitting device 1 to emit the first output light 12 and the second output light 13 alternately in time.

### [Seventh embodiment]

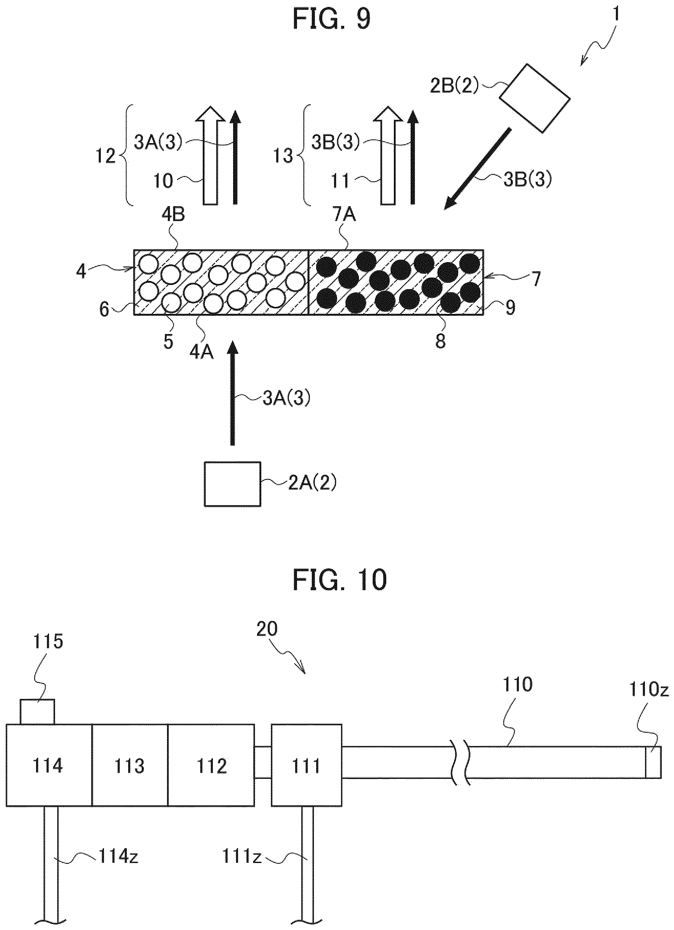

Next, a light emitting device 1 according to a seventh embodiment is described with reference to FIG. 9. The same components as those of the above-described embodiment are denoted by the same reference numerals, and redundant descriptions are omitted.

As illustrated in FIG. 9, in the light emitting device 1 according to the present embodiment, a primary light 3A is emitted to a front 4A of a first wavelength converter 4, and a first wavelength-converted light 10 is emitted from a back 4B of the first wavelength converter 4. A primary light 3B is emitted to a front 7A of a second wavelength converter 7, and a second wavelength-converted light 11 is emitted from the front 7A of the second wavelength converter 7. In these points, the light emitting device 1 according to the present embodiment is different from the light emitting device 1 according to the first embodiment.

In the present embodiment, as illustrated in FIG. 9, a light source 2 includes a first light source 2A and a second light source 2B. The first light source 2A emits the primary light 3A, and the second light source 2B emits the primary light 3B. In the present embodiment, the primary light 3A for exciting the first phosphor 5 and the primary light 3B for exciting the second phosphor 8 have different optical axes, and the primary light 3A and the primary light 3B are emitted alternately in time.

Next, the operation of the light emitting device 1 according to the present embodiment is described. As illustrated in FIG. 9, in the light emitting device 1 according to the present embodiment, first, the first wavelength converter 4 and the second wavelength converter 7 are respectively irradiated with the primary light 3A emitted by the first light source 2A and the primary light 3B emitted by the second light source 2B alternately in time. The primary light 3A is emitted to the front 4A of the first wavelength converter 4, most of the emitted primary light 3A enters the first wavelength converter 4 through the front 4A, and the remaining part is reflected on the surface of the first wavelength converter 4. Then, when the primary light 3A passes through the first wavelength converter 4, the first phosphor 5 included in the first wavelength converter 4 absorbs a part of the primary light 3A to emit the first wavelength-converted light 10. The first wavelength-converted light 10 is emitted from the back 4B of the first wavelength converter 4. In contrast, the primary light 3B is emitted to the front 7A of the second wavelength converter 7, most of the emitted primary light 3B enters the second wavelength converter 7 through the front 7A, and the remaining part is reflected on the surface of the second wavelength converter 7. In the second wavelength converter 7, the second phosphor 8 excited by the primary light 3 emits the second wavelength-converted light 11, and the second wavelength-converted light 11 is emitted from the front 7A. In this way, the first light source 2A and the second light source 2B repeatedly turn on and off alternately in time, which enables the light emitting device 1 to emit the first output light 12 and the second output light 13 alternately in time.

In the light emitting device 1 according to the present embodiment, the positions of the first wavelength converter 4 and the second wavelength converter 7 may be changed. That is, the primary light 3A may be emitted to the front 4A of the first wavelength converter 4, and the first wavelength-converted light 10 may be emitted from the front 4A of the first wavelength converter 4. The primary light 3B may be emitted to the front 7A of the second wavelength converter 7, and the second wavelength-converted light 11 may be emitted from the back 7B of the second wavelength converter 7.

Also in the present embodiment, by changing the positions of the first wavelength converter 4 and the second wavelength converter 7 with respect to the light source 2, the first output light 12 and the second output light 13 may be emitted alternately in time.

The light emitting device 1 according to the present embodiment has been described using the light emitting devices according to the first to seventh embodiments. The above-described light emitting device 1 may be used for medical purposes. That is, the light emitting device 1 may be a medical light emitting device. In other words, the light emitting device 1 may be a medical illumination device. Such a light emitting device 1 is advantageous in diagnosing disease state because it achieves the coexistence of normal observation and special observation as described above.

The light emitting device 1 may be used for optical coherence tomography (OCT) or the like. However, preferably, the light emitting device 1 is used for either a fluorescence imaging method or photodynamic therapy. The light emitting device 1 used in these methods is a light emitting device for a medical system using a drug, such as a fluorescent drug or a photosensitive drug. These methods are a promising medical technology with a wide range of applications and are highly practical. The light emitting device 1 illuminates the inside of the living body with a broad near-infrared high-output light through the "living body window" and makes the fluorescent drug or photosensitive drug taken into the living body fully functional, which is expected to have a large therapeutic effect.

The fluorescence imaging method is a method of observing a lesion by administering a fluorescent drug that selectively binds to the lesion, such as a tumor, to a subject, exciting the fluorescent drug with a specific light, and detecting and imaging fluorescence emitted from the fluorescent drug with an image sensor. The fluorescence imaging method makes it possible to observe lesions that are difficult to observe using only general illumination. As the fluorescent drug, a drug that absorbs excitation light in the near-infrared range, and emits fluorescence in the near-infrared range and at a wavelength longer than the excitation light is usable. Examples of the fluorescent drug used include at least one selected from the group consisting of indocyanine green (ICG), a phthalocyanine-based compound, a talaporfin sodium-based compound, and a dipicolylcyanine (DIPCY)-based compound.

The photodynamic therapy is a treatment method of administering a photosensitive drug that selectively binds to a target biological tissue to a subject and irradiating the photosensitive drug with near-infrared light. When the photosensitive drug is irradiated with the near-infrared light, the photosensitive drug generates active oxygen, which is usable to treat lesions, such as tumors or infections. Examples of the photosensitive drug used include at least one selected from the group consisting of a phthalocyanine-based compound, a talaporfin sodium-based compound, and a porfimer sodium-based compound.

The light emitting device 1 according to the present embodiment may be used as a light source for a sensing system or an illumination system for a sensing system. With the light emitting device 1, an orthodox light receiving element having light receiving sensitivity in the near-infrared wavelength range may be used to configure a high-sensitivity sensing system. This provides a light emitting device that facilitates miniaturization of the sensing system and broadening of the sensing range.

### [Healthcare system]

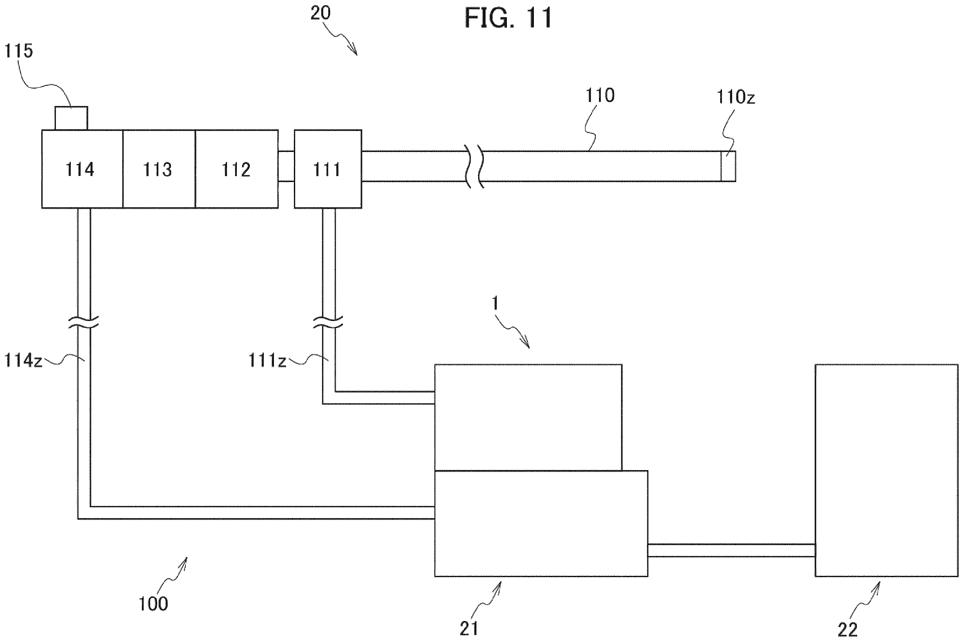

Next, a medical system including the above-described light emitting device 1 is described. Specifically, as an example of the medical system, an endoscope 20 provided with the light emitting device 1 and an endoscope system 100 using the endoscope 20 are described with reference to FIGS. 10 and 11.

### (Endoscope)

As illustrated in FIG. 10, the endoscope 20 according to the present embodiment includes the above-described light emitting device 1. The endoscope 20 includes a scope 110, a light source connector 111, a mount adapter 112, a relay lens 113, a camera head 114, and an operation switch 115.

The scope 110 is an elongated light guide member capable of guiding light from end to end and is inserted into the body when in use. The scope 110 includes an imaging window 110z at its tip. For the imaging window 110z, an optical material, such as optical glass or optical plastic, is used. The scope 110 includes an optical fiber for guiding light introduced from the light source connector 111 to the tip, and an optical fiber for transmitting an optical image that enters through the imaging window 110z.

The light source connector 111 introduces illumination light emitted to a diseased part and the like in the body from the light emitting device 1. In the present embodiment, the illumination light includes visible light and near-infrared light. The light introduced into the light source connector 111 is guided to the tip of the scope 110 through the optical fiber to be emitted to a diseased part and the like in the body from the imaging window 110z. As illustrated in FIG. 10, the light source connector 111 is provided with a transmission cable 111z for guiding illumination light from the light emitting device 1 to the scope 110. The transmission cable 111z may include an optical fiber.

The mount adapter 112 is a member for mounting the scope 110 on the camera head 114. Various scopes 110 are detachably mountable on the mount adapter 112.

The relay lens 113 converges the optical image transmitted through the scope 110 on the imaging surface of the image sensor. The relay lens 113 may be moved in accordance with the operation amount of the operation switch 115 to perform focus adjustment and magnification adjustment.

The camera head 114 includes a color separation prism inside. The color separation prism separates the light converged by the relay lens 113 into four colors of R light (red light), G light (green light), B light (blue light), and IR light (near-infrared light). The color separation prism includes, for example, a light transmitting member, such as glass.

The camera head 114 further includes an image sensor as a detector inside. For example, four image sensors are provided, and each of the four image sensors converts an optical image formed on an imaging surface into an electric signal. The image sensor is not limited, but at least one of CCD (Charge Coupled Device) or CMOS (Complementary Metal Oxide Semiconductor) is usable. The four image sensors are dedicated sensors for receiving light of the IR component (near-infrared component), the R component (red component), the G component (green component), and the B component (blue component), respectively.

The camera head 114 may have a color filter inside instead of the color separation prism. The color filter is provided on the imaging surface of the image sensor. For example, four color filters are provided, and the four color filters receive the light converged by the relay lens 113 and selectively transmit R light (red light), G light (green light), B light (blue light), and IR light (near-infrared light), respectively.

Preferably, the color filter for selectively transmitting IR light includes a barrier film for cutting the reflection component of near-infrared light (IR light) included in the illumination light. This enables only the fluorescence composed of IR light emitted from a fluorescent drug, such as ICG, to form an image on the imaging surface of the image sensor for IR light. Therefore, the diseased part luminous with the fluorescent drug is easily observed clearly.

As illustrated in FIG. 10, a signal cable 114z is connected to the camera head 114 to transmit an electric signal from the image sensor to a CCU 21 described later.

In the endoscope 20 having such a configuration, light from a subject is guided to the relay lens 113 through the scope 110 and is further transmitted through the color separation prism in the camera head 114 to form images on the four image sensors.

As illustrated in FIG. 11, the endoscope system 100 includes the endoscope 20 for imaging the inside of a subject, a CCU (Camera Control Unit) 12, and a display device 22, such as a display.

The CCU 21 includes at least an RGB signal processing unit, an IR signal processing unit, and an output unit. The CCU 21 executes a program stored in the internal or external memory of the CCU 21 to realize the respective functions of the RGB signal processing unit, the IR signal processing unit, and the output unit.

The RGB signal processing unit converts the R component, G component, and B component electrical signals from the image sensors into video signals that are displayable on the display device 22 and outputs the video signals to the output unit. The IR signal processing unit converts the IR component electrical signal from the image sensor into a video signal and outputs the video signal to the output unit.

The output unit outputs at least one of the video signals of respective RGB color components or the video signal of the IR component to the display device 22. For example, the output unit outputs video signals on the basis of either a simultaneous output mode or a superimposed output mode.

In the simultaneous output mode, the output unit simultaneously outputs the RGB image and the IR image on separate screens. The simultaneous output mode enables the diseased part to be observed by comparing the RGB image and the IR image on the separate screens. In the superimposed output mode, the output unit outputs a composite image in which the RGB image and the IR image are superimposed. The superimposed output mode enables the diseased part luminous with the ICG to be clearly observed, for example in the RGB image.

The display device 22 displays an image of an object, such as a diseased part, on a screen on the basis of video signals from the CCU 21. In the simultaneous output mode, the display device 22 divides the screen into multiple screens and displays the RGB image and the IR image side by side on each screen. In the superimposed output mode, the display device 22 displays a composite image in which an RGB image and an IR image are superimposed on each other on a single screen.

Next, functions of the endoscope 20 and the endoscope system 100 according to the present embodiment are described. When a subject is observed using the endoscope system 100, first, indocyanine green (ICG) as a fluorescent substance is administered to the subject. As a result, ICG accumulates at a site of lymph, tumor, or the like (diseased part).

Next, visible light and near-infrared light are introduced from the light emitting device 1 to the light source connector 111 through the transmission cable 111z. The light introduced into the light source connector 111 is guided to the tip side of the scope 110 and projected from the imaging window 110z to be emitted to the diseased part and the periphery of the diseased part. The light reflected from the diseased part and the like and the fluorescence emitted from the ICG are guided to the rear end side of the scope 110 through the imaging window 110z and the optical fiber, converged by the relay lens 113 to enter into the color separation prism inside the camera head 114.

In the color separation prism, among the incident light, the light of the IR component separated by the IR separation prism is imaged as an optical image of an infrared component by the IR light image sensor. The light of the R component separated by the red separation prism is imaged as an optical image of the red component by the R light image sensor. The light of the G component separated by the green separation prism is imaged as an optical image of the green component by the G light image sensor. The light of the B component separated by the blue separation prism is imaged as an optical image of the blue component by the B light image sensor.

The electrical signal of the IR component converted by the IR light image sensor is converted into a video signal by the IR signal processing unit in the CCU 21. The electric signals of the R component, G component, and B component converted by the RGB light image sensors are converted into respective video signals by the RGB signal processing unit in the CCU 21. The image signal of the IR component, and the image signals of the R component, G component, and B component in synchronization with each other are output to the display device 22.

When the simultaneous output mode is set in the CCU 21, the RGB image and the IR image are simultaneously displayed on two screens on the display device 22. When the superimposed output mode is set in the CCU 21, a composite image in which the RGB image and the IR image are superimposed is displayed on the display device 22.

As described above, the endoscope 20 according to the present embodiment includes the light emitting device 1. Therefore, by efficiently exciting the fluorescent drug to emit light using the endoscope 20, the diseased part is clearly observed.

Preferably, the endoscope 20 according to the present embodiment further includes a detector for detecting fluorescence emitted from a fluorescent drug that has absorbed the first wavelength-converted light 10. By providing the endoscope 20 with the detector for detecting fluorescence emitted from a fluorescent drug in addition to the light emitting device 1, the diseased part is specified only by the endoscope. This makes it possible to perform medical examination and treatment with less burden on the patient, since there is no need to open the abdomen wide to identify the diseased part as in the conventional method. This also enables the doctor using the endoscope 20 to accurately identify the diseased part, which improves the efficiency of treatment.

As described above, preferably, the medical system is used for either the fluorescence imaging method or photodynamic therapy. The medical system used in these methods is a promising medical technology with a wide range of applications and is highly practical. The medical system illuminates the inside of the living body with a broad near-infrared high-output light through the "living body window" and makes the fluorescent drug or photosensitive drug taken into the living body fully functional, which is expected to have a large therapeutic effect. Further, such a medical system uses the light emitting device 1 having a relatively simple configuration, which is advantageous in reducing the size and the cost.

### [Electronic apparatus]

Next, an electronic apparatus according to the present embodiment is described. The electronic apparatus according to the present embodiment includes a light emitting device 1. As described above, the light emitting device 1 is expected to have a large therapeutic effect, and it is easy to miniaturize the sensing system. Since the electronic apparatus according to the present embodiment uses the light emitting device 1, when it is used for a medical device or a sensing device, a large therapeutic effect, miniaturization of the sensing system, and the like are expected.

The electronic apparatus includes, for example, the light emitting device 1, and a light receiving element. The light receiving element is, for example, a sensor, such as an infrared sensor for detecting light in a near-infrared wavelength range. The electronic apparatus may be any of an information recognition device, a sorting device, a detection device, or an inspection device. As described above, these devices also facilitate miniaturization of the sensing system and broadening of the sensing range.

The information recognition device is, for example, a driver support system that recognizes the surrounding situation by detecting reflected components of emitted infrared rays.

The sorting device is, for example, a device that sorts an irradiated object into predetermined categories by using the difference in infrared light components between the irradiation light and reflected light reflected by the irradiated object.

The detection device is, for example, a device that detects a liquid. Examples of liquids include water, and flammable liquids that are prohibited from being transported in aircraft. Specifically, the detection device may be a device for detecting moisture adhering to glass, and moisture absorbed by an object, such as sponge or fine powder. The detection device may visualize the detected liquid. Specifically, the detection device may visualize the distribution information of the detected liquid.

The inspection device may be any of a medical inspection device, an agricultural and livestock inspection device, a fishery inspection device, or an industrial inspection device. These devices are useful for inspecting an inspection object in each industry.

The medical inspection device is, for example, an examination device that examines the health condition of a human or non-human animal. Non-human animals are, for example, domestic animals. The medical inspection device is, for example, a device used for a biological examination, such as a fundus examination or a blood oxygen saturation examination, and a device used for examination of an organ, such as a blood vessel or an organ. The medical inspection device may be a device for examining the inside of a living body or a device for examining the outside of a living body.

The agricultural and livestock inspection device is, for example, a device for inspecting agricultural and livestock products including agricultural products and livestock products. Agricultural products may be used as foods, for example, fruits and vegetables, or cereals, or as fuels, such as oils. Livestock products include, for example, meat and dairy products. The agricultural and livestock inspection device may be a device for non-destructively inspecting the inside or outside of the agricultural and livestock products. Examples of the agricultural and livestock inspection device includes a device for inspecting the sugar content of vegetables and fruits, a device for inspecting the acidity of vegetables and fruits, a device for inspecting the freshness of vegetables and fruits by the visualization of leaf veins, a device for inspecting the quality of vegetables and fruits by the visualization of wounds and internal defects, a device for inspecting the quality of meat, and a device for inspecting the quality of processed foods processed with milk, meat, or the like as raw materials.

The fishery inspection device is, for example, a device for inspecting the flesh quality of fish, such as tuna, or a device for inspecting the presence or absence of the contents in shells of shellfish.

The industrial inspection device is, for example, a foreign matter inspection device, a content inspection device, a condition inspection device, or a structure inspection device.

Examples of the foreign matter inspection device include a device for inspecting foreign matter in a liquid contained in a container, such as a beverage or a liquid medicine, a device for inspecting foreign matter in a packaging material, a device for inspecting foreign matter in a printed image, a device for inspecting foreign matter in a semiconductor or an electronic component, a device for inspecting foreign matter, such as residual bone in food, dust, or machine oil, a device for inspecting foreign matter in processed food in a container, and a device for inspecting foreign matter in medical devices, such as adhesive plasters, medical and pharmaceutical products, or quasi-drugs.

Examples of the content inspection device include a device for inspecting the content of a liquid contained in a container, such as a beverage or a liquid medicine, a device for inspecting the content of a processed food contained in a container, and a device for inspecting the content of asbestos in building materials.

Examples of the state inspection device include a device for inspecting packaging state of a packaging material, and a device for inspecting printing state of a packaging material.

Examples of the structure inspection device include an internal non-destructive inspection device and an external non-destructive inspection device for a composite member or a composite component, such as a resin product. A specific example of the resin product is, for example, a metal brush with a part of metal wire embedded in the resin, and the inspection device inspects the bonding state of the resin and the metal.

The electronic apparatus may use color night vision technology. Color night vision technology uses a correlation of reflection intensity between visible light and infrared rays to colorize an image by assigning infrared rays to RGB signals for each wavelength. According to the color night vision technology, a color image is obtained only by infrared rays, and it is particularly suitable for a security device.

As described above, the electronic apparatus includes the light emitting device 1. When the light emitting device 1 includes a power source, a light source 2, a first wavelength converter 4, and a second wavelength converter 7, it is not necessary to accommodate all of them in one housing. Therefore, the electronic apparatus according to the present embodiment provides a highly accurate and compact inspection method, or the like, with excellent operability.

### [Inspection method]

Next, an inspection method according to the present embodiment is described. As described above, the electronic apparatus including the light emitting device 1 is also usable as an inspection device. That is, the light emitting device 1 is usable in the inspection method according to the present embodiment. This provides a highly accurate and compact inspection method with excellent operability.

### Examples

The light emitting device according to the present embodiment is described below in more detail with reference to examples, but the present embodiment is not limited thereto.

### [Preparation of phosphor]

### (First phosphor)

A first phosphor was synthesized using a preparation method utilizing a solid phase reaction. The Cr³⁺-activated phosphor used in the first phosphor is an oxide phosphor represented by a formula: (Gd_{0.75}La_{0.25})₃(Ga_{0.97}Cr_{0.03})₂Ga₃O₁₂. In synthesizing the first phosphor, the following compound powders were used as main raw materials.
Gadolinium oxide (Gd₂O₃): purity 3N, Wako Pure Chemical Corporation
Lanthanum oxide (La₂O₃): purity 3N, Wako Pure Chemical Corporation
Gallium oxide (Ga₂O₃): purity 4N, Wako Pure Chemical Corporation
Chromium oxide (Cr₂O₃): Purity 3N, Kojundo Chemical Laboratory Co.,Ltd.

First, the above-described raw materials were weighed to obtain a compound of a stoichiometric composition (Gd_{0.75}La_{0.25})₃(Ga_{0.97}Cr_{0.03})₂Ga₃O₁₂. The weighed raw materials were then put into a beaker containing pure water and stirred with a magnetic stirrer for 1 hour. Thus, a slurry-like mixed raw material of the pure water and raw materials was obtained. Then, the slurry-like mixed raw material was dried entirely using a dryer. The mixed raw material after drying was pulverized using a mortar and a pestle to obtain a calcined raw material.

The above-described calcined raw material was transferred to a small alumina crucible and calcined in air at 1400 °C to 1500 °C for 1 hour in a box-type electric furnace to obtain the phosphor of this example. The temperature rise and fall rate was set at 400 °C/h. The body color of the obtained phosphor was light green.

### (Second phosphor)

A commercially available YAG phosphor (Y₃Al₂Al₃O₁₂:Ce³⁺) was obtained as a second phosphor. In consideration of the fluorescence peak wavelength and the like, the chemical composition of the YAG phosphor is estimated to be (Y_{0.97}Ce_{0.03})₃Al₂Al₃O₁₂.

### [Evaluation]

### (Crystal structure analysis)

The crystal structures of the first phosphor and the second phosphor were evaluated using an X-ray diffraction apparatus (X`Pert PRO; manufactured by Spectris Co., Ltd., PANalytical).

As a result of evaluation, it was found that the first and second phosphors were mainly made from compounds with a garnet crystal structure, although the details are omitted. That is, both the first phosphor and the second phosphor were found to be garnet phosphors.

### (Fluorescence spectrum)

Next, wavelength converters including the first phosphor and the second phosphor were prepared, and the fluorescence properties were evaluated. Specifically, as for the first phosphor, the wavelength converter was produced by sintering the above-described calcined raw material, which was formed into a pellet shape using a hand press, in air at 1450 °C for 1 hour. As for the second phosphor, the wavelength converter was produced by calcining the phosphor formed into a pellet shape by hand press under a reducing atmosphere of 1600 °C to 1700 °C for 1 to 6 hours. Then, the obtained wavelength converters were excited by a laser light, and the fluorescence spectra of the fluorescence emitted from the wavelength converters were evaluated. At this time, the central wavelength of the laser light was set at 445 nm. The energy of the laser light was set to 3.87 W.

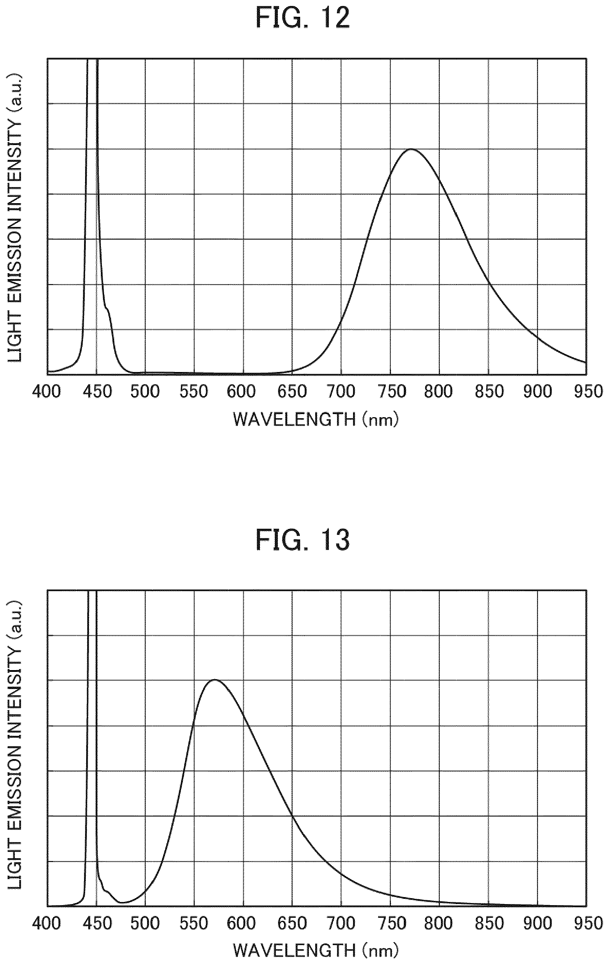

FIG. 12 illustrates the fluorescence spectrum of the first phosphor. The fluorescence spectrum of the first phosphor was formed from a broad spectrum determined to be attributed to the d-d transition of Cr³⁺. The fluorescence spectrum of the first phosphor had a light component over the entire wavelength range of 700 nm or more to 800 nm or less. In addition, the fluorescence spectrum of the first phosphor had a peak where the intensity shows a maximum value in a wavelength range of 700 nm or more. Specifically, the peak wavelength of the fluorescence spectrum of the first phosphor was 768 nm.

FIG. 13 illustrates the fluorescence spectrum of the second phosphor. The fluorescence spectrum of the second phosphor was formed from a broad spectrum determined to be attributed to the 5d¹→4f¹ transition of Ce³⁺. The fluorescence spectrum of the second phosphor had a peak where the fluorescence intensity shows a maximum value within a wavelength range of 380 nm or more to less than 700 nm. Specifically, the peak wavelength of the fluorescence spectrum of the second phosphor was 570 nm.

For example, a phosphor wheel separately coated with the first phosphor and the second phosphor is manufactured, and these phosphors are excited by a laser while the phosphor wheel is rotated. Then, from the above results, it is said that a light emitting device is realized in which near-infrared light emitted by the first phosphor and visible light emitted by the second phosphor are outputted alternately in time.

### Industrial Applicability

In accordance with the present disclosure, there is provided a light emitting device that emits near-infrared light and visible light to obtain a high-contrast observation result, and a medical system, an electronic apparatus, and an inspection method using the light emitting device.

### Reference Signs List

- 1: Light emitting device
- 2: Light source
- 3: Primary light
- 4: First wavelength converter
- 5: First phosphor
- 7: Second wavelength converter
- 8: Second phosphor
- 10: First wavelength-converted light
- 11: Second wavelength-converted light
- 12: First output light
- 13: Second output light

## Claims

1. A light emitting device (1), comprising:
a light source (2) configured to emit a primary light (3);
a first wavelength converter (4) comprising a first phosphor (5) that absorbs the primary light (3) and converts the primary light (3) into a first wavelength-converted light (10) having a wavelength longer than that of the primary light (3); and
a second wavelength converter (7) comprising a second phosphor (8) that absorbs the primary light (3) and converts the primary light (3) into a second wavelength-converted light (11) having a wavelength longer than that of the primary light (3), wherein
the first wavelength-converted light (10) is a fluorescence having a light component over an entire wavelength range of 700 nm or more to 800 nm or less and having a peak where a fluorescence intensity shows a maximum value in a wavelength range of 700 nm or more;
the second wavelength-converted light (11) is a fluorescence having a peak where a fluorescence intensity shows a maximum value in a wavelength range of 380 nm or more to less than 700 nm; and
the light emitting device (1) emits a first output light (12) including the first wavelength-converted light (10) and a second output light (13) including the second wavelength-converted light (11) alternately in time.

2. The light emitting device (1) according to claim 1, wherein the primary light (3) is a laser light.

3. The light emitting device (1) according to claim 1 or claim 2, wherein the primary light (3) for exciting the first phosphor (5) and the primary light (3) for exciting the second phosphor (8) have different optical axes from each other.

4. The light emitting device (1) according to claim 1 or claim 2, wherein the primary light (3) for exciting the first phosphor (5) and the primary light (3) for exciting the second phosphor (8) are emitted by the light source (2).

5. The light emitting device (1) according to claim 4, wherein the primary light (3) for exciting the first phosphor (5) and the primary light (3) for exciting the second phosphor (8) have a same optical axis.

6. The light emitting device (1) according to any one of claims 1 to 5, wherein the light emitting device (1) emits the first output light (12) and the second output light (13) alternately in time as the first wavelength converter (4) and the second wavelength converter (7) change positions relative to the light source (2).

7. The light emitting device (1) according to any one of claims 1 to 6, wherein the first phosphor (5) is activated with a transition metal ion.

8. The light emitting device (1) according to any one of claims 1 to 7, wherein the second phosphor (8) is activated with at least one of Ce³⁺ or Eu²⁺.

9. The light emitting device (1) according to any one of claims 1 to 8, wherein a mixed light of the primary light (3) and the second wavelength-converted light (11) has a correlated color temperature of 2500 K or more to less than 7000 K.

10. The light emitting device (1) according to any one of claims 1 to 9, wherein the light emitting device (1) is a light source (2) for a sensing system, or an illumination system for a sensing system.

11. The light emitting device (1) according to any one of claims 1 to 10, wherein the light emitting device (1) is used in either a fluorescence imaging method or a photodynamic therapy.

12. A medical system, comprising:
the light emitting device (1) according to any one of claims 1 to 11.

13. An electronic apparatus, comprising:
the light emitting device (1) according to any one of claims 1 to 10.

14. The electronic apparatus according to claim 13, wherein the electronic apparatus is any one of an information recognition device, a sorting device, a detection device, or an inspection device.

15. An inspection method, comprising:
using the light emitting device (1) according to any one of claims 1 to 10.

## Patentansprüche

1. Lichtemittierende Vorrichtung (1), aufweisend:
eine Lichtquelle (2), die konfiguriert ist, ein Primärlicht (3) zu emittieren;
einen ersten Wellenlängenkonverter (4), der einen ersten Leuchtstoff (5) aufweist, der das Primärlicht (3) absorbiert und das Primärlicht (3) in ein erstes wellenlängenumgewandeltes Licht (10) mit einer längeren Wellenlänge als das Primärlicht (3) umwandelt; und
einen zweiten Wellenlängenkonverter (7), der einen zweiten Leuchtstoff (8) aufweist, der das Primärlicht (3) absorbiert und das Primärlicht (3) in ein zweites wellenlängenumgewandeltes Licht (11) mit einer längeren Wellenlänge als das Primärlicht (3) umwandelt, wobei
das erste wellenlängenumgewandelte Licht (10) eine Fluoreszenz ist, die eine Lichtkomponente über einen gesamten Wellenlängenbereich von 700 nm oder mehr bis 800 nm oder weniger aufweist und einen Peak aufweist, bei dem eine Fluoreszenzintensität einen Maximalwert in einem Wellenlängenbereich von 700 nm oder mehr aufweist;
das zweite wellenlängenumgewandelte Licht (11) eine Fluoreszenz mit einem Peak ist, bei dem eine Fluoreszenzintensität einen Maximalwert in einem Wellenlängenbereich von 380 nm oder mehr bis weniger als 700 nm aufweist; und
die lichtemittierende Vorrichtung (1) ein erstes Ausgangslicht (12), welches das erste wellenlängenumgewandelte Licht (10) aufweist, und ein zweites Ausgangslicht (13), welches das zweite wellenlängenumgewandelte Licht (11) aufweist, zeitlich abwechselnd emittiert.

2. Lichtemittierende Vorrichtung (1) nach Anspruch 1, wobei das Primärlicht (3) ein Laserlicht ist.

3. Lichtemittierende Vorrichtung (1) nach Anspruch 1 oder 2, wobei das Primärlicht (3) zur Anregung des ersten Leuchtstoffs (5) und das Primärlicht (3) zur Anregung des zweiten Leuchtstoffs (8) voneinander unterschiedliche optische Achsen aufweisen.

4. Lichtemittierende Vorrichtung (1) nach Anspruch 1 oder 2, wobei das Primärlicht (3) zur Anregung des ersten Leuchtstoffs (5) und das Primärlicht (3) zur Anregung des zweiten Leuchtstoffs (8) durch die Lichtquelle (2) emittiert werden.

5. Lichtemittierende Vorrichtung (1) nach Anspruch 4, wobei das Primärlicht (3) zur Anregung des ersten Leuchtstoffs (5) und das Primärlicht (3) zur Anregung des zweiten Leuchtstoffs (8) eine selbe optische Achse aufweisen.

6. Lichtemittierende Vorrichtung (1) nach einem der Ansprüche 1 bis 5, wobei die lichtemittierende Vorrichtung (1) das erste Ausgangslicht (12) und das zweite Ausgangslicht (13) in dem Maße zeitlich abwechselnd emittiert, in dem der erste Wellenlängenkonverter (4) und der zweite Wellenlängenkonverter (7) ihre Positionen relativ zu der Lichtquelle (2) ändern.

7. Lichtemittierende Vorrichtung (1) nach einem der Ansprüche 1 bis 6, wobei der erste Leuchtstoff (5) mit einem Übergangsmetallion aktiviert wird.

8. Lichtemittierende Vorrichtung (1) nach einem der Ansprüche 1 bis 7, wobei der zweite Leuchtstoff (8) mit Ce³⁺ und/oder Eu²⁺ aktiviert wird.

9. Lichtemittierende Vorrichtung (1) nach einem der Ansprüche 1 bis 8, wobei ein Mischlicht aus dem Primärlicht (3) und dem zweiten wellenlängenumgewandelten Licht (11) eine korrelierte Farbtemperatur von 2500 K oder mehr bis weniger als 7000 K aufweist.

10. Lichtemittierende Vorrichtung (1) nach einem der Ansprüche 1 bis 9, wobei die lichtemittierende Vorrichtung (1) eine Lichtquelle (2) für ein Erfassungssystem oder ein Beleuchtungssystem für ein Erfassungssystem ist.

11. Lichtemittierende Vorrichtung (1) nach einem der Ansprüche 1 bis 10, wobei die lichtemittierende Vorrichtung (1) entweder in einem Fluoreszenz-Bildgebungsverfahren oder in einer fotodynamischen Therapie verwendet wird.

12. Medizinisches System, aufweisend:
die lichtemittierende Vorrichtung (1) nach einem der Ansprüche 1 bis 11.

13. Elektronische Einrichtung, aufweisend:
die lichtemittierende Vorrichtung (1) nach einem der Ansprüche 1 bis 10.

14. Elektronische Einrichtung nach Anspruch 13, wobei die elektronische Einrichtung eine Informationserkennungsvorrichtung, eine Sortiervorrichtung, eine Detektionsvorrichtung oder eine Inspektionsvorrichtung ist.

15. Inspektionsverfahren, umfassend:
Verwenden der lichtemittierenden Vorrichtung (1) nach einem der Ansprüche 1 bis 10.

## Revendications

1. Dispositif électroluminescent (1), comprenant :
une source de lumière (2) configurée pour émettre une lumière primaire (3) ;
un premier convertisseur de longueur d'onde (4) comprenant un premier luminophore (5) qui absorbe la lumière primaire (3) et convertit la lumière primaire (3) en une première lumière convertie en longueur d'onde (10) ayant une longueur d'onde plus longue que celle de la lumière primaire (3) ; et
un second convertisseur de longueur d'onde (7) comprenant un second luminophore (8) qui absorbe la lumière primaire (3) et convertit la lumière primaire (3) en une seconde lumière convertie en longueur d'onde (11) ayant une longueur d'onde plus longue que celle de la lumière primaire (3), dans lequel
la première lumière convertie en longueur d'onde (10) est une fluorescence ayant un composant de lumière sur toute une plage de longueurs d'onde de 700 nm ou plus à 800 nm ou moins et ayant un pic où une intensité de fluorescence présente une valeur maximale dans une plage de longueurs d'onde de 700 nm ou plus ;
la seconde lumière convertie en longueur d'onde (11) est une fluorescence ayant un pic où une intensité de florescence présente une valeur maximale dans une plage de longueurs d'onde de 380 nm ou plus à moins de 700 nm ; et
le dispositif électroluminescent (1) émet une première lumière de sortie (12) incluant la première lumière convertie en longueur d'onde (10) et une seconde lumière de sortie (13) incluant la seconde lumière convertie en longueur d'onde (11) alternativement dans le temps.

2. Dispositif électroluminescent (1) selon la revendication 1, dans lequel la lumière primaire (3) est une lumière laser.

3. Dispositif électroluminescent (1) selon la revendication 1 ou la revendication 2, dans lequel la lumière primaire (3) destinée à exciter le premier luminophore (5) et la lumière primaire (3) destinée à exciter le second luminophore (8) présentent des axes optiques différents l'un de l'autre.

4. Dispositif électroluminescent (1) selon la revendication 1 ou la revendication 2, dans lequel la lumière primaire (3) destinée à exciter le premier luminophore (5) et la lumière primaire (3) destinée à exciter le second luminophore (8) sont émises par la source de lumière (2).

5. Dispositif électroluminescent (1) selon la revendication 4, dans lequel la lumière primaire (3) destinée à exciter le premier luminophore (5) et la lumière primaire (3) destinée à exciter le second luminophore (8) ont un même axe optique.

6. Dispositif électroluminescent (1) selon l'une quelconque des revendications 1 à 5, dans lequel le dispositif électroluminescent (1) émet la première lumière de sortie (12) et la seconde lumière de sortie (13) alternativement dans le temps lorsque le premier convertisseur de longueur d'onde (4) et le second convertisseur de longueur d'onde (7) changent de positions par rapport à la source de lumière (2).

7. Dispositif électroluminescent (1) selon l'une quelconque des revendications 1 à 6, dans lequel le premier luminophore (5) est activé avec un ion de métal de transition.

8. Dispositif électroluminescent (1) selon l'une quelconque des revendications 1 à 7, dans lequel le second luminophore (8) est activé avec au moins l'un de Ce³⁺ ou Eu²⁺.

9. Dispositif électroluminescent (1) selon l'une quelconque des revendications 1 à 8, dans lequel une lumière mélangée de la lumière primaire (3) et de la seconde lumière convertie en longueur d'onde (11) présente une température de couleur corrélée de 2500 K ou plus à moins de 7000 K.

10. Dispositif électroluminescent (1) selon l'une quelconque des revendications 1 à 9, dans lequel le dispositif électroluminescent (1) est une source de lumière (2) pour un système de détection, ou un système d'éclairage pour un système de détection.

11. Dispositif électroluminescent (1) selon l'une quelconque des revendications 1 à 10, dans lequel le dispositif électroluminescent (1) est utilisé soit dans un procédé d'imagerie de fluorescence, soit dans une thérapie photodynamique.

12. Système médical, comprenant :
le dispositif électroluminescent (1) selon l'une quelconque des revendications 1 à 11.

13. Appareil électronique, comprenant :
le dispositif électroluminescent (1) selon l'une quelconque des revendications 1 à 10.

14. Appareil électronique selon la revendication 13, dans lequel l'appareil électronique est l'un quelconque d'un dispositif de reconnaissance d'informations, d'un dispositif de tri, d'un dispositif de détection ou d'un dispositif d'inspection.

15. Procédé d'inspection, comprenant :
l'utilisation du dispositif électroluminescent (1) selon l'une quelconque des revendications 1 à 10.
